# EUROPEAN PATENT APPLICATION

(11) **EP 4 101 428 A1**
(43) Date of publication of application: **14.12.2022**
(21) Application number: 21179023.3
(22) Date of filing: 11.06.2021
(51) Int. Cl.: A61F 13/42, A61B 5/11

(54) **SYSTEM AND METHOD FOR MONITORING A PATIENT WEARING AN ABSORBENT ARTICLE**

(71) Applicant: AssistMe GmbH, 10555 Berlin (DE)
(72) Inventor: Iyengar, Vijay, 14167 Berlin (DE); Biglands, Victoria, 12059 Berlin (DE)
(74) Representative: Gulde & Partner

(57) **Abstract**

A sensor module (1) for an absorbent article, the sensor module (1) comprising: a first sensor unit (11) positioned on or in the absorbent article and configured to detect a signal dependent on presence of liquid in the absorbent article; a second sensor unit (12) positioned on or in the absorbent article and configured to detect a spatial orientation of the absorbent article; a transmitter (20) connected to the first sensor unit (11) and the second sensor unit (12) and configured to transmit the signal provided by the first sensor unit (11) and the spatial orientation provided by the second sensor unit (12) to an external device (30). A corresponding system is also disclosed.

## Description

### Field of the Invention

The present invention relates to a system and a method for monitoring a patient wearing an absorbent article, particularly to systems, methods, sensors and a Graphical User Interface (GUI) that are used for detecting and documenting a current orientation of a resident wearing an absorbent article and providing information if there is a risk of leakage detected for a present or upcoming orientation of the resident wearing the absorbent article.

### Technological Background

One of the biggest challenges that modern societies might face in the coming years is the aging of their populations. The number of caregivers and elderly at nursing homes will become less balanced, therefore less caregivers will attend more residents.

Already today caregivers do check on residents according to a tight time schedule, not only making it an exhausting workload but also rendering it difficult to determine, which urgent need to attend first. Also important to note is that caregivers have assigned a certain number of residents they have to monitor on each shift, which renders efficient scheduling even more important.

Community care is increasingly interested in using interactive technology to improve the outcomes and management of pressure ulcers affecting elderly people. For example bedsores may be prevented by frequently repositioning the affected persons to avoid stress of resident's skin. However, it already is a burden for caregivers to document the frequently required repositioning events for big groups of affected persons. At the same time, confirming the effectiveness of frequent repositioning is an important goal, in order to ensure that the standard of care is appropriate and because labor costs associated with this intervention are considerable.

The problem of repositioning residents is further related to monitoring of absorbent articles worn by the residents. In particular, improvements are needed with respect to the monitoring of such absorbent articles in relation to repositioning of residents.

### Description of the Invention

The objective of the invention is solved and the disadvantages of the prior art are overcome by the subject-matter of the present disclosure, i.e., by a sensor module for an absorbent article, by a mobile device for monitoring a patient wearing an absorbent article, by a system comprising the sensor module according to the present disclosure and the mobile device according to the present disclosure and a method for monitoring a patient wearing an absorbent article having the sensor module according to the present disclosure using the mobile device according to the present disclosure.

In a first aspect of the present invention, a sensor module for an absorbent article is provided, the sensor module comprising: a first sensor unit positioned on or in the absorbent article and configured to detect a signal dependent on presence of liquid in the absorbent article; a second sensor unit positioned on or in the absorbent article and configured to detect a spatial orientation of the absorbent article; a transmitter connected to the first sensor unit and the second sensor unit and configured to transmit the signal provided by the first sensor unit and the spatial orientation provided by the second sensor unit to an external device.

In the context of the present disclosure, a location and a spatial orientation of an object, particularly of the absorbent article, together fully describe how the given object, particularly the absorbent article, is placed in space. In other words, a spatial orientation defines how an object, particularly the absorbent article, is placed in the space it occupies, while a location defines where the object, particularly the absorbent article, is placed in space accordingly.

The present disclosure relates to a wearable absorbent article that is configured to indicate, by means of the sensor module, in which orientation the person (resident) wearing the absorbent article is positioned. The spatial orientation may be determined by means of a gyroscope, a magnetometer and/or an accelerometer. The sensor module may output raw data or process the raw data to determine one of predefined orientations such as a left lateral orientation or a right lateral orientation or a supine orientation or a prone orientation or a sitting orientation. Therefore, raw data and/or processed data may be output by the sensor module.

The transmitter may be any suitable radio communication transmitter such as a Bluetooth or ZigBee communication transmitter/receiver (transceiver). However, the transmitter may also be a WLAN transceiver or a transceiver configured for 3G/4G/5G communication.

The sensor module may further comprise a controller. The sensor module may be realized using dedicated components or custom-made FPGA or ASIC circuits. The sensor module comprises necessary common components such as a data bus communicatively coupled to a memory or a power supply such as a battery. Additionally, other components of the sensor module are communicatively coupled to the system bus so that they may be managed by the controller. Such common modules of computer systems are typical in the technical field concerned and will be evident to one skilled in the art. For the sake of brevity, details of such common modules of computer systems are omitted herein.

In an embodiment, the sensor module interprets the orientation of the person (resident) based on the gravity, the earth's magnetic field. The controller of the sensor module may be configured to perform such an interpretation of the raw data. The sensor module may further be configured to send these processed signals to an external node, an edge, or a cloud system. In an example, the raw data comprises an acceleration of the absorbent article in time with respect to the direction of the gravity, gyroscopic motion with respect to how fast the person is rotating in 3D, and magnetometer earth magnetic field 3D compass data. According to this example, the raw data are processed (interpreted) into an orientation, particularly into a 3D rotational orientation of the absorbent article correlated with a spatial orientation of the body of a person wearing the absorbent article.

The transmitter of the present sensor module may be further configured to receive configuration data and/or transmission of detected events as opposed to raw data transmission only.

Preferably, the sensor module further comprises a location reporting module. The location reporting module may be based on a Global Positioning System (GPS) or similar systems or based on a local positioning system (LPS) that is a navigation system providing location information anywhere within a coverage of a network, where there typically is received a signal from three or more signaling beacons positioned at known locations.

Preferably, the sensor module is such that the location reporting module further comprises: (A) a third sensor unit positioned on or in the absorbent article and configured to detect a location of the absorbent article, wherein the transmitter is further connected to the third sensor unit and configured to transmit the location provided by the third sensor unit to the external device or (B) a location beacon signal transmitter remote of the absorbent article.

Option (A) above concerns GPS or LPS signals being received for determining location whereas option (B) concerns a case where the sensor module itself transmits, for example broadcasts, a beacon signal to be interpreted by other receivers.

According to an embodiment, a network of transmitters is installed in a care home. In a coarse positioning method, a mobile node may receive beacon signals that identify residents' rooms, in which the beacons are located. The user's (or otherwise caregiver's) mobile node may obtain floor plan information relating to the building and estimate beacon locations based on the floor plan and beacon signal strength. This allows to seamlessly and automatically determine when a particular user (i.e. user's mobile device/node is able to determine its location e.g. room 1 based on the received beacon signals and the floor plan) actually changes an orientation of a particular resident (resident's are preferably assigned to rooms e.g. resident 1 assigned to room 1) or performs another task in the vicinity of the resident.

Favorably, the controller of the sensor module, according to any embodiments given above, is further configured to receive a plurality of spatial orientations from the second sensor unit, to determine whether a difference between received spatial orientations exceeds a predetermined threshold, and, in case the predetermined threshold is exceeded, to control the transmitter to transmit a signal indicative of a change of the spatial orientation of the absorbent article to the external device.

For example, every four hours a resident must change orientation. If a change of orientation by +/- 15° or more is considered as a repositioning, a final orientation has to be more than the +/- 15° different from an initial orientation.

Preferably, the sensor module according to any one of the embodiments given above, is such that the first sensor unit is further configured to detect an amount of liquid in the absorbent article and wherein the signal output by the first sensor unit indicates said amount.

The detected amount may be an absolute amount or a relative amount. The amount of liquid may be used to determine a level or volume of liquid, a fulness, and/or a remaining capacity of the corresponding absorbent article.

Favorably, the sensor module according to any one of the preceding embodiments, is such that the first sensor unit is configured for electrical impedance spectroscopy, EIS, measurements.

Further preferred, the signal dependent on presence of liquid in the absorbent article may be received from a capacitive moisture sensor, CMS, attached to the absorbent article as the first sensor. The CMS may be configured to induce an electrical signal to the absorbent article, and to measure a capacitance of the absorbent article and a liquid content thereof. The capacitance measurement can be translated to a digital signal output that can be subsequently received and analyzed. In this context, attaching the CMS to an absorbent article may not only concern attaching the CMS to the external side of the absorbent article but also mean positioning the CMS within the article i.e. embedding a CMS within the article. Fastening or mounting techniques such as gluing, stitching, or the like may be employed.

The electrical signal detected by the CMS may not only be dependent on the presence of a liquid as such in the absorbent article but is preferably also be dependent on the amount of the liquid that is present in the absorbent article. Such expected signals will typically be dependent on the absorbent article and thus a configuration step will preferably involve a process of testing a given absorbent article in order to define its associated CMS response.

For example, an absorbent article is tested for its maximum capacity by submerging it in a tank of liquid and measuring the maximum liquid intake. Additionally, there may be also measured an operational capacity by introducing liquid (in a manner close to real use-case situation) into the article and detecting leakage at a point of maximum operational capacity. The maximum operational capacity may be different than the maximum capacity. The maximum operational capacity is thereafter correlated with a specific sensor signal response. A reference signal value for an empty absorbent article is also determined. Correspondingly, signal values at different remaining capacity levels may be read and stored for reference. Alternatively, it may be assumed that the signal obtained from the sensor changes in a relatively linear fashion from the set first value indicating an empty absorbent article to the set second value indicating a fullness at operational capacity of the absorbent article. When a linear approach is defined, the respective thresholds may be determined automatically. Such tested values may be made available as the predefined reporting configuration, which is used by the incontinence detection method.

A second aspect of the present disclosure relates to a mobile device for monitoring a patient wearing an absorbent article, the mobile device comprising: a receiver configured to receive manifold data from the sensor module according to any one of the embodiments given above, the received data comprising a signal indicative of presence of liquid in an absorbent article and a spatial orientation of the absorbent article; a controller connected to the receiver and configured to determine a leakage risk for the absorbent article based on the received data and to control an output means based on the determination to output leakage risk information to a user.

Further, a controller or a processor of the mobile device may be configured to process the received orientation data to establish a time of a last orientation change and a type of the last orientation and to process the received signal dependent on presence of liquid in the absorbent article in order to establish an amount of liquid in the absorbent article.

Preferably, the mobile device further comprises a memory storing first information associating spatial orientations of the absorbent article and liquid amounts in the absorbent article with leakage probabilities, wherein the signal indicative of presence of liquid in an absorbent article is indicative of an amount of liquid in the absorbent article, and wherein the controller is configured to determine the leakage risk based on the received data and the stored first information.

Preferably, the first information is determined in a test stand and may be stored as a look-up-table or a fitted parametrized function or the like wherein information on a type of the absorbent article is linked with information associating each of at least two orientations with a leakage probability value at a given amount of liquid in the absorbent article.

Each of the aforementioned at least two orientations may be selected from a group comprising: left lateral orientations, right lateral orientations, supine orientations, prone orientations, sitting orientations. A plural form is used herein, for example when referring to left lateral orientations, due to a fact that several left lateral orientations may be identified differing by respective angles within a predefined range of angles for example +/- 10 degrees. In other words there may be several variants of orientations generally falling under a definition of a left lateral orientation. The same approach may be applied to the remaining groups of orientations. Other orientations may also apply such as a recovery orientations (also called semi-prone) or intermediate orientations between the orientations identified above.

For example, a given absorbent article has a high risk of leakage when a person wearing the absorbent article is positioned in a left lateral orientation while the absorbent article is at least 90% full. Further, the same absorbent article has a high risk of leakage when a person wearing the absorbent article is positioned in a supine orientation while the absorbent article is at least 95% full. Additionally, the same absorbent article has a high risk of leakage when a person wearing the absorbent article is positioned in a sitting orientation while the absorbent article is at least 98% full. Therefore, a leakage risk may be also different at the same amount of liquid in the absorbent article depending on an orientation of the absorbent article.

The memory may further store third information on a urination pattern of the person wearing the absorbent article. Such urination pattern may depend on gender, weight of a person. Additionally the urination pattern may be correlated with an amount of liquid in a single event as well as with a frequency of such events. Based on such third information on a urination pattern, the amount of liquid present in the absorbent article may be estimated based on the urination pattern of the (kind of) user wearing the absorbent article. In an embodiment the first information may comprise the third information.

Favorably, the mobile device of any of the abovementioned examples may further comprise a memory storing second information related to a time interval for repositioning a person wearing the absorbent article, wherein the controller is configured to determine a repositioning need based on the second information, to predict a leakage risk related to the repositioning need based on the received data and to control an output means based on the prediction to output predicted leakage risk information to the user.

The second information is preferably associated with a person and a type of an associated absorbent article worn by the person. The second information together with the first information and/or the third information allows to provide a user with detailed information on a time of last repositioning of a particular patient, a need for a further repositioning and a signal indicative of a probable leakage.

Particularly, the repositioning-related information may be output by a user's device when the predefined interval has elapsed wherein the repositioning-related information preferably comprises information on a next orientation and when the leakage probability value associated with the next orientation is equal or over a predefined threshold, user's device may be configured to further output a signal indicative of a probable leakage. In an exemplary embodiment, for safety reasons, repositioning is recommended at least every six hours for adults at risk, and every four hours for adults at high risk.

Preferably, the mobile device of the embodiment given above, is such that the second information specifies a repositioning sequence and the controller is configured to predict the leakage risk for an upcoming spatial orientation according to the repositioning sequence and/or wherein the controller is configured to predict the leakage risk for the spatial orientations included in the first information based on the received data and to determine an upcoming spatial orientation with reduced leakage risk for the repositioning and wherein the controller is configured to control the output means to output the upcoming spatial orientation to the user. The mobile device according to this embodiment may advantageously suggest a next orientation of a person based on the current orientation, wherein the suggested next orientation results in lowest probability of leakage of the absorbent article worn by the person.

In another embodiment, a sequence of orientations may be determined that will result in a prolonged usage time of the absorbent article based on a urination pattern and a leakage probability of the absorbent article in relation to different orientations, e.g., determined based on the first information as described above. For example, when the absorbent article is relatively empty, it may be suggested that the patient takes an orientation where leakage possibility is relatively high (i.e. an amount of liquid resulting in a high risk of leakage is low when compared to other orientations) as the first orientation and then gradually change orientations to the orientations where leakage possibility is relatively low (i.e. an amount of liquid resulting in a high risk of leakage is high when compared to other orientations). Taking the embodiment given above referring to 90%, 95% and 98% it could be recommended that the person starts from the left lateral orientation (90%), is then moved to the supine orientation (95%) and is then moved to the sitting orientation (98%).

In other words, when starting with a new absorbent article it is preferred to apply the orientations in a sequence in which a parameter of fulness (e.g., a relative or absolute amount of liquid) at which there is a high risk of leakage gradually increases from the lowest (90% in the example) to the highest (98% in the example).

In order to maintain a quality of care delivery, a care home's workflows preferably include the need of documenting every change of orientation, and for this documentation the user's identification is needed each time when a repositioning is reported. Favorably, the mobile device (such as a tablet or a smartphone) of any of the preceding embodiments, hence further comprises an input means, wherein the controller is further configured to prompt a user to input a confirmation of repositioning of a resident.

If a repositioning is determined as being made by a user, meaning for example that a vicinity between the sensor module location and a user's mobile node is detected and that a repositioning schedule indicated a due repositioning for around the actual time, a GUI of a mobile device may display an input data screen where an electronic signature is needed in order to certify a time, an orientation and an identity user of the repositioning action. Therein, the time, orientation and identity of the user may be automatically be determined based on a communication signal, the signal provided by the second sensor unit or the controller and a signal transmitted by the user's mobile node, respectively.

In such cases a GUI displayed by the mobile device of a user may prompt the user to approve an overview of the recent repositioning event and provide a GUI canvas, in which the user may sign to confirm and document the repositioning. Similarly, the user may be given an opportunity to cancel the repositioning event so that it may be executed again. The information confirmed by the user may comprise a time of detection of a change of orientation, a date, the new orientation and the like. Therein, the information may be based on data received from the sensor units. The event may be confirmed with a password or with biometric data of the user or with an electronic signature. The user may be given an opportunity to alter some information regarding the repositioning

Preferably, the user is prompted in response to determining that the received data is indicative of a change of spatial difference between received spatial orientations that exceeds a predetermined threshold and/or if a location of the user's mobile device is determined to be in predetermined proximity to a location of the absorbent article.

This feature allows to automatically associate a given absorbent article and its wearer with a particular user so that documenting repositioning events may be more automatic. A user may be given feedback whether a change of orientation of the absorbent article meets predefined one or more threshold(s). It may also be verified whether the new orientation corresponds to a orientation recommended by the present system.

Yet another aspect of the present disclosure relates to a system comprising the sensor module according to the present disclosure and the mobile device according to the present disclosure. In other embodiments there may be a plurality of sensor modules according to the present disclosure and correspondingly a plurality of mobile devices according to the present disclosure.

A further aspect of the present invention relates to a method for monitoring a patient wearing an absorbent article having the sensor module according to the present disclosure using the mobile device according to the present disclosure. In other embodiments there may be a plurality of sensor modules according to the present disclosure and a plurality of mobile devices according to the present disclosure.

Another aspect of the present invention relates to a computer program comprising program code means for performing all the steps of the computer-implemented method for monitoring a patient wearing an absorbent article, according to the present invention, when said program is run on a computer.

Another aspect of the present invention relates to a computer readable medium storing computer-executable instructions performing all the steps of the computer-implemented method for monitoring a patient wearing an absorbent article, according to the present disclosure, when executed on a computer.

Such computer programs are typically executed by utilizing the computing resources in a computing device. Applications are stored on a non-transitory computer-readable medium. An example of a non-transitory medium is a non-volatile memory, for example a flash memory while an example of a volatile memory is RAM. The computer instructions are executed by a suitable processor. These memories are exemplary recording media for storing computer programs comprising computer-executable instructions performing all the steps of the computer-implemented method according the technical concept presented herein.

Typically an absorbent article will be manufactured as including the sensor module according to the present disclosure. This elevates a burden of properly placing the system on/in the absorbent article from caretakers (users). However, in other embodiments, a caretaker may equip an off-the-shelf absorbent article with the sensor module. In such a case, the controller of the sensor module may be programmed with the predefined reporting configuration tailored for this specific absorbent article. Such a configuration may be made available to users via a database and/or an online tool or a software application for mobile devices.

Also, a person of skill in the art should recognize that the functionality of various computing devices may be combined or integrated into a single computing device, or the functionality of a particular computing device may be distributed across one or more other computing devices without departing from the scope of the embodiments of the present invention.

Further aspects of the present invention are disclosed in the dependent claims or the following description of the drawings.

### Brief Description of the Drawings

Features will become apparent to those of ordinary skill in the art by describing in detail exemplary embodiments with reference to the attached drawings in which:
Figure 1 illustrates a schematic drawing of the sensor module according to an embodiment of the present disclosure;
Figure 2 presents a top level overview of the method according to an embodiment of the present disclosure;
Figure 3 shows a diagram of an operation of the system according to an embodiment of the present disclosure, specifically before and after a change of orientation is made;
Figure 4 is a diagram further illustrating how the system according to an embodiment of the present disclosure performs;
Figure 5 depicts a diagram showing content of a display of a mobile device according to an embodiment of the present disclosure and a notification about a risk of leakage corresponding to a next orientation that a user may receive;
Figure 6 further illustrates a diagram illustrating a process of repositioning documentation according to an embodiment of the method according to the disclosure; and
Figure 7 shows a diagram describing an approach and steps to be taken in order to measure the risk of leakages corresponding to a next orientation, based on personas and test rig models.

### Detailed Description of the Invention

Reference will now be made in detail to embodiments, examples of which are illustrated in the accompanying drawings. Effects and features of the exemplary embodiments, and implementation methods thereof will be described with reference to the accompanying drawings. In the drawings, like reference numerals denote like elements, and redundant descriptions are omitted. The present invention, however, may be embodied in various different forms, and should not be construed as being limited to only the illustrated embodiments herein. Rather, these embodiments are provided as examples so that this disclosure will be thorough and complete, and will fully convey the aspects and features of the present invention to those skilled in the art.

Accordingly, processes, elements, and techniques that are not considered necessary to those having ordinary skill in the art for a complete understanding of the aspects and features of the present invention may not be described. In the drawings, the relative sizes of elements, layers, and regions may be exaggerated for clarity.

As used herein, the term "and/or" includes any and all combinations of one or more of the associated listed items. Further, the use of "may" when describing embodiments of the present invention refers to "one or more embodiments of the present invention." In the following description of embodiments of the present invention, the terms of a singular form may include plural forms unless the context clearly indicates otherwise.

It will be understood that although the terms "first" and "second" are used to describe various elements, these elements should not be limited by these terms. These terms are only used to distinguish one element from another element. For example, a first element may be named a second element and, similarly, a second element may be named a first element, without departing from the scope of the present invention. Expressions such as "at least one of," when preceding a list of elements, modify the entire list of elements and do not modify the individual elements of the list.

As used herein, the term "substantially," "about," and similar terms are used as terms of approximation and not as terms of degree, and are intended to account for the inherent deviations in measured or calculated values that would be recognized by those of ordinary skill in the art. Further, if the term "substantially" is used in combination with a feature that could be expressed using a numeric value, the term "substantially" denotes a range of +/- 5% of the value centered on the value.

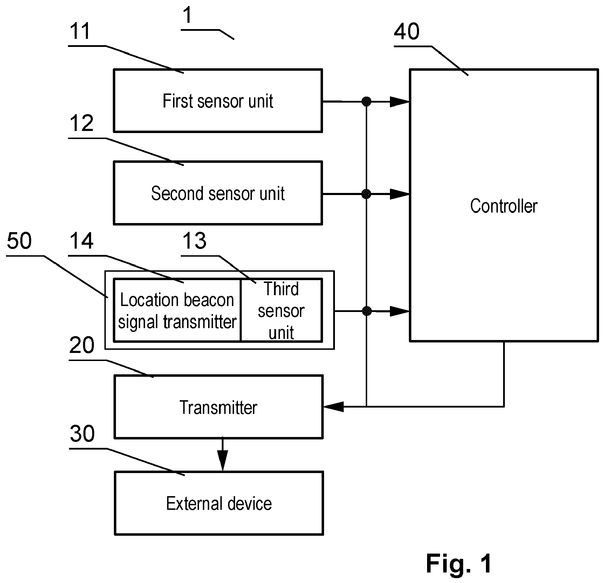

Figure 1 illustrates a schematic drawing of a sensor module according to an embodiment of the present disclosure. A sensor module 1 (add a dashed box or the like around the sensor module for making clear what belongs to it, e.g., the external device does not) for an absorbent article, comprises a first sensor unit 11 positioned on or in the absorbent article and configured to detect a signal dependent on presence of liquid in the absorbent article and a second sensor unit 12 positioned on or in the absorbent article and configured to detect a spatial orientation of the absorbent article.

Further, the sensor module 1 comprises a transmitter 20 connected to the first sensor unit 11 and the second sensor unit 12 and configured to transmit the signal provided by the first sensor unit 11 and the spatial orientation provided by the second sensor unit 12 to an external device 30.

The sensor module 1 may further comprise a location reporting module 50 wherein the location reporting module 50 may further comprise a third sensor unit 13 positioned on or in the absorbent article and configured to detect a location of the absorbent article, wherein the transmitter 20 is further connected to the third sensor unit 13 and configured to transmit the location provided by the third sensor unit 13 to the external device 30. Alternatively, the location reporting module 50 may further comprise a location beacon signal transmitter 14. A signal transmitted by the location beacon signal transmitter 14 may be received by one or more receivers deployed in a care facility in order to determine location of the particular sensor module 1.

The sensor module 1 may further comprise a controller 40 connected to the first sensor unit 11, the second sensor unit 12 and the transmitter 20.The controller 40 being configured to receive a plurality of spatial orientations from the second sensor unit 12, to determine whether a difference between received spatial orientations exceeds a predetermined threshold, and, in case the predetermined threshold is exceeded, to control the transmitter 20 to transmit a signal indicative of a change of the spatial orientation of the absorbent article to the external device 30.

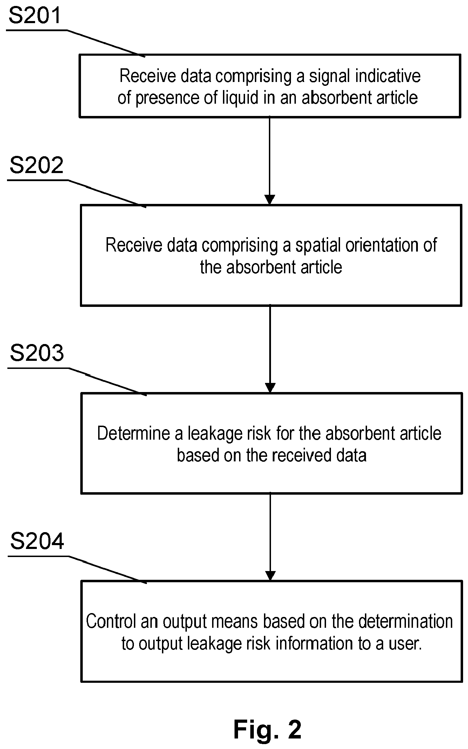

Figure 2 presents a top-level overview of the method according to an embodiment of the present disclosure. The method may be implemented by a mobile device for monitoring a patient wearing an absorbent article. A suitable receiver may be configured to receive manifold data from a sensor module according to the present disclosure, the received data comprising a signal S201 indicative of presence of liquid in an absorbent article and a spatial orientation S202 of the absorbent article. Further a controller connected to the receiver may be configured to determine S203 a leakage risk for the absorbent article, based on the received data and to control S204 an output means based on the determination to output the determined leakage risk information to a user.

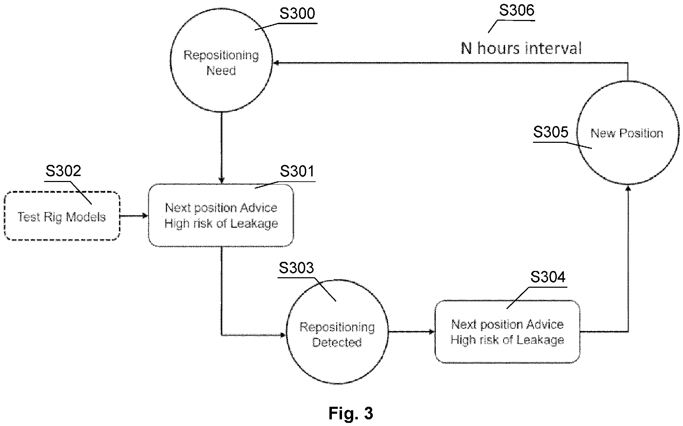

Figure 3 shows a diagram of an operation of the system according to an embodiment of the present invention, specifically before and after a change of orientation is made. Particularly, the process allows to timely reposition a person being cared for and document the fact of repositioning.

For example at step S300 a resident at risk of ulcer injuries is determined to need a change of orientation. Such determined need of change of orientation and also information about a risk of leakage in the next recommended orientation is provided and displayed S301 to a user for example by means of a GUI of a mobile device. The user will, based on this information, take a decision regarding which care approach to take in the circumstances. In order to indicate a risk of leakage, a person and the aforementioned first information S302 that correctly adapts to the profile of the resident (e.g. weight, orientation, absorbent Article) are taken into account.

At step S303 a change of orientation is detected by the sensor module (for example a change of orientation more than +/- 15° or other threshold value in one or more axes). This change is made by the user.

After the change is detected in step S305, which may be compared to the recommended orientation in step S304, the users preferably need to document and authenticate that the care need was delivered correctly. For this a signature is preferably needed, which may be received via the mobile GUI and a suitable GUI control for an electronic signature. Alternatively, biometric data of a user may be used as such a signature. The user signs and the documentation is updated and recorded on a backend.

The resident's new orientation in step S305 will preferably reactivate a timer for N hours until a next repositioning. After that interval is over, the system will preferably notify the user that a new orientation is needed. As an option, an unattended need for repositioning may be signaled to another user or a supervisor.

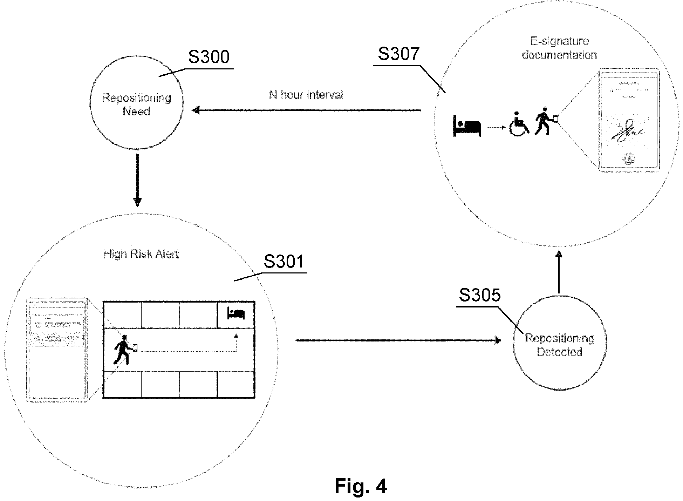

Figure 4 is a diagram further illustrating how the system according to an embodiment of the present disclosure performs. Steps S300, S301, S305 and S307 are schematically shown. A user may be prompted at step S301 with location information of a particular resident while the N-hour interval may be resident-dependent in view of medical staff recommendations made based on resident's condition. In addition or alternatively the N-hour interval could also depend on weight of a resident as it may be beneficial to reposition heavier residents more frequently than lighter residents. Step S307 introduces a further option of E-signature-based documentation of the repositioning detected at step S305.

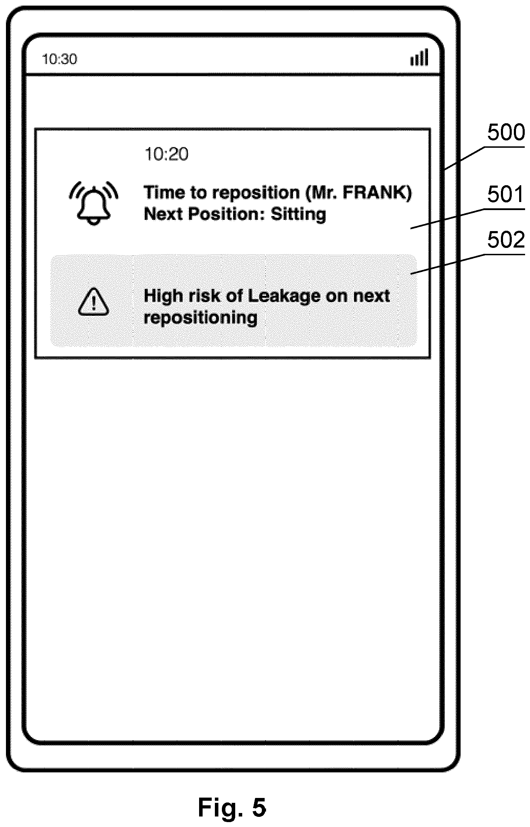

Figure 5 depicts a diagram showing a display of a mobile device according to an embodiment of the present disclosure and a notification about a risk of leakage corresponding to a next orientation that a user may receive to apply to a resident.

A mobile device's GUI comprising a push notification on a locked screen is shown in the preferred example 500. Such a push notification to the user's smartphone may describe, which resident is concerned and what is the next recommended orientation of the resident 501. A time stamp may be given indicating when the need was first triggered. A risk of leakage assessment in the next orientation 502 may be given to support decision making of the user.

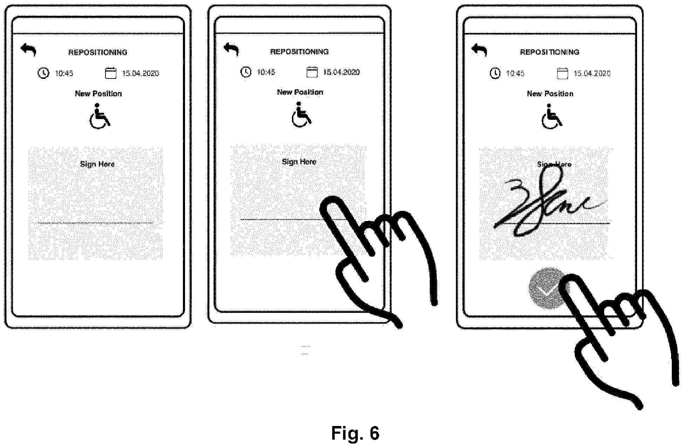

Figure 6 illustrates a diagram illustrating a process of repositioning documentation according to an embodiment of the method according to the disclosure. This diagram depicts a flow of an electronic signature in order to document a repositioning need and its addressing accordingly. When a new change of orientation is detected (as a result of a user moving the resident) a GUI may be configured to prompt the user with an overview of the recent repositioning event and a GUI canvas in which the user may sign. The user may go back on the event, for example to correct a resident's orientation, or cancel the event. A displayed repositioning report may comprise a time of detection of the new orientation and/or a date and/or a GUI Canvas to sign on. In particular, a handwritten signature may be acceptable, which is further confirmed by pressing a GUI button.

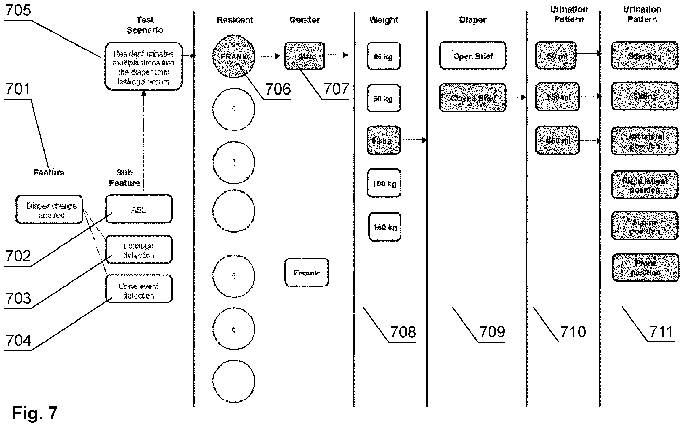

Figure 7 shows a diagram describing an approach and steps to be taken in order to measure the risk of leakages corresponding to a next orientation, based on personas and test rig models.

In order to test different scenarios for different orientations e.g. various walking, seating and sleeping orientations in relation to different persons characterized by parameters such as body weight, age and gender, it is very time consuming and costly to carry out actual testing of different absorbent articles on humans. As a result, there is a need to design and perform an automated test rig, which is configured to determine and output all required data for different scenarios. In order to illustrate how this process allows to determine whether there is a risk of leakage of an absorbent article, an example of a resident "Frank" 706 is presented who is a resident with low mobility suffering from incontinence.

A typical absorbent article such as a diaper is estimated to hold between 600 to 1000ml of urine before leaking. In order to know if there is a risk of leakage, a test rig is used to test the sensor module as well as the absorbent article in different circumstances. The sensor module validation tests preferably involve simulating human urination using the test-rig to quantify the effect of using the sensor module (such as based on an IDE inter digitated electrode as explained above by way of example related to the CMS) and wearable system to detect moisture in a diaper. The test-rig preferably rotates a mannequin, applies pressure to the mannequin, and distributes synthetic urine through a heating a pumping fluid-mechanical system.

The diagram relates to a feature 701 of urine detection. The test rig is able to simulate different levels of urine detection that may be defined as follows: ABL (maximum absorption capacity) 702; leakage 703, first urine event detection 704. One test scenario 705 is: a resident urinates multiple times on a diaper until a leakage occurs.

The first information 706, which may be referred to as a residents' model, corresponding to the first information, is defined by taking into account parameters such as a gender 707, weight 708, type of absorbent product 709 (for example a particular retail product), the third information on urination pattern 710: e.g. 50ml (e.g. below average); 150ml (e.g. an approximated average); 450ml (e.g. above average) (in an example a urination pattern may be simplified by assuming one urination soon after repositioning and then lack of urination for a predefined time such as 2 hours. Alternatively the amount may concern a total expected liquid amount in the predefined time such as 2 hours), different orientations 711 the resident can be in, all the predefined orientations 711 are tested with diapers at full capacity in order to know, which of the orientations of the resident has a higher chance of leakage.

As indicated above, presence of the third information in the first information is optional as such third information may be separate from the first information. For each combination or parameters of the first information 706 to 711 there is determined a leakage probability, which is additionally stored in the first information.

In addition to the full capacity, other predefined capacities may be tested, for example 90%, 95%, 98%. Results of such tests may be stored in the first information 706. This is particularly useful since although a theoretical capacity may be different than full i.e. 100%, the given absorbent article may still leak in a non-optimum orientation e.g. at 95% capacity used. For a more specific example, in a sitting orientation a 95% capacity does not cause a high leakage probability while the same capacity of the same absorbent article may cause a high leakage probability while being orientated in a prone orientation considering for example a person's weight of 100kg.

### Reference signs

- 1: sensor module
- 11: first sensor unit
- 12: second sensor unit
- 20: transmitter
- 30: external device
- 50: location reporting module
- 13: third sensor unit
- 14: location beacon signal transmitter
- S201 to S204: Method steps
- S300 to S307: Method steps
- 500: GUI notification example
- 501: GUI notification on a next recommended orientation of the resident
- 502: GUI notification on a risk of leakage

## Claims

1. A sensor module (1) for an absorbent article, the sensor module (1) comprising:
a first sensor unit (11) positioned on or in the absorbent article and configured to detect a signal dependent on presence of liquid in the absorbent article;
a second sensor unit (12) positioned on or in the absorbent article and configured to detect a spatial orientation of the absorbent article;
a transmitter (20) connected to the first sensor unit (11) and the second sensor unit (12) and configured to transmit the signal provided by the first sensor unit (11) and the spatial orientation provided by the second sensor unit (12) to an external device (30).

2. The sensor module (1) according to claim 1, further comprising a location reporting module (50).

3. The sensor module (1) according to claim 2, wherein the location reporting module (50) further comprises:
a third sensor unit (13) positioned on or in the absorbent article and configured to detect a location of the absorbent article, wherein the transmitter (20) is further connected to the third sensor unit (13) and configured to transmit the location provided by the third sensor unit (13) to the external device (30) or;
a location beacon signal transmitter (14).

4. The sensor module (1) according to any one of the preceding claims, further comprising a controller (40) connected to the second sensor unit (12) and the transmitter (20), the controller (40) being configured to receive a plurality of spatial orientations from the second sensor unit (12), to determine whether a difference between received spatial orientations exceeds a predetermined threshold, and, in case the predetermined threshold is exceeded, to control the transmitter (20) to transmit a signal indicative of a change of the spatial orientation of the absorbent article to the external device (30).

5. The sensor module (1) according to any one of the preceding claims, wherein the first sensor unit (11) is further configured to detect an amount of liquid in the absorbent article and wherein the signal by the first sensor unit (11) indicates said amount.

6. The sensor module (1) according to any one of the preceding claims, wherein the first sensor unit (11) is configured for electrical impedance spectroscopy, EIS, measurements and/or the second sensor unit comprises one of an accelerometer, a gyroscope and a magnetometer.

7. A mobile device for monitoring a patient wearing an absorbent article, the mobile device comprising:
a receiver configured to receive manifold data from a sensor module according to any one of the claims 1 to 6, the received data comprising a signal indicative of presence of liquid in an absorbent article and a spatial orientation of the absorbent article;
a controller connected to the receiver and configured to determine a leakage risk for the absorbent article based on the received data and to control an output means based on the determination to output leakage risk information to a user.

8. The mobile device of claim 7, further comprising a memory storing first information associating spatial orientations of the absorbent article and liquid amounts in the absorbent article with leakage probabilities, wherein the signal indicative of presence of liquid in an absorbent article is indicative of an amount of liquid in the absorbent article, and wherein the controller is configured to determine the leakage risk based on the received data and the stored first information.

9. The mobile device of claim 7 or 8, further comprising a memory storing second information related to a time interval for repositioning a person wearing the absorbent article, wherein the controller is configured to determine a repositioning need based on the second information, to predict a leakage risk related to the repositioning need based on the received data and to control an output means based on the prediction to output predicted leakage risk information to the user.

10. The mobile device of claims 8 and 9, wherein the second information specifies a repositioning sequence and the controller is configured to predict the leakage risk for an upcoming spatial orientation according to the repositioning sequence and/or wherein the controller is configured to predict the leakage risk for the spatial orientations included in the first information based on the received data and to determine an upcoming spatial orientation with reduced leakage risk for the repositioning and wherein the controller is configured to control the output means to output the upcoming spatial orientation to the user.

11. The mobile device of any of claims 7 to 10, further comprising an input means, wherein the controller is further configured to prompt the user to input a confirmation of repositioning.

12. The mobile device of claim 11, wherein the user is prompted in response to that the received data is indicative of a change of spatial difference between received spatial orientations that exceeds a predetermined threshold and/or if a location of the mobile device is determined to be in predetermined proximity to a location of the absorbent article.

13. A system comprising a sensor module according to any one of the claims 1 to 6 and a mobile device according to any one of claims 7 to 12.

14. A method for monitoring a patient wearing an absorbent article having a sensor module according to any one of the claims 1 to 6 using a mobile device according to any one of claims 7 to 12.

15. A computer-readable medium comprising instructions which, when executed by a computer, cause the computer to carry out the steps of the method of claim 13.
